# EUROPEAN PATENT APPLICATION

(11) **EP 4 755 415 A1**
(43) Date of publication of application: **10.06.2026**
(21) Application number: 23948188.0
(22) Date of filing: 07.09.2023
(51) Int. Cl.: A61M 5/158, A61M 5/50

(54) **INDWELLING NEEDLE**

(30) Priority: 04.08.2023 CN 202310975977
(71) Applicant: WTTOD US Inc., Dover County Of Kent, Delaware 19901 (US); Lu, Jun, Wuxi, Jiangsu 214400 (CN)
(72) Inventor: LU, Jun, Wuxi, Jiangsu 214400 (CN)
(74) Representative: Bardehle Pagenberg S.L.
(86) International application number: PCT/CN2023/117365
(87) International publication number: WO 2025/030621

(57) **Abstract**

Disclosed is an indwelling needle. The indwelling needle includes a catheter hub and a needle hub, wherein a catheter is provided at a head end of the catheter hub, and an opening is provided in a tail end of the catheter hub; a needle tubing is provided at a head end of the needle hub, and a vent plug and a tail end cap are provided at a tail end of the needle hub. The indwelling needle further includes a safety protection cover, wherein a needle tip protection housing is provided in the safety protection cover, and an isolation plug is provided at a head end of the needle tip protection housing. In a factory default state, the safety protection cover is sleeved at the tail end of the catheter hub, the needle hub is sleeved on the safety protection cover, the needle tip protection housing is inserted into the catheter hub, an inner cavity of the catheter hub is sealed by means of the isolation plug, and the needle tubing passes through the needle tip protection housing and then extends into the catheter; and in a needle withdrawn state, a needle tip of the needle tubing is withdrawn into the needle tip protection housing, the needle tip protection housing is pulled out of the catheter hub, the safety protection cover is separated from the catheter hub, and the tail end cap is connected to the tail end of the catheter hub in a sealed manner.

## Description

The present application claims the priority of the Chinese Patent Application No. 202310975977.4, filed on August 4, 2023 to Chinese Patent Office, which is incorporated herein by reference in its entirety.

### TECHNICAL FIELD

The present application relates to the technical field of medical devices, for example, to an indwelling needle.

### BACKGROUND ART

An intravenous indwelling needle is an infusion tool, which is usually composed of a stainless steel needle core, a soft outer cannula and a plastic needle hub. During puncture, the outer cannula and the needle core are punctured into a blood vessel together, and then the needle core is withdrawn, leaving the outer cannula in the blood vessel. For patients undergoing long-term infusion or intermittent or continuous infusion, the intravenous indwelling needle can avoid damages and complications caused by repeated puncture, and is thus widely used in clinical practice.

### SUMMARY OF THE INVENTION

The present application discloses an indwelling needle. The indwelling needle includes a catheter hub and a needle hub, wherein a catheter is provided at a head end of the catheter hub, and an opening is provided in a tail end of the catheter hub; a needle tubing is provided at a head end of the needle hub, and a vent plug and a tail end cap are provided at a tail end of the needle hub. The indwelling needle further includes a safety protection cover, wherein a needle tip protection housing is provided in the safety protection cover, and an isolation plug is provided at a head end of the needle tip protection housing. In a factory default state, the safety protection cover is sleeved at the tail end of the catheter hub, the needle hub is sleeved on the safety protection cover, the needle tip protection housing is inserted into the catheter hub, an inner cavity of the catheter hub is sealed by means of the isolation plug, and the needle tubing passes through the needle tip protection housing and then extends into the catheter; and in a needle withdrawn state, a needle tip of the needle tubing is withdrawn into the needle tip protection housing, the needle tip protection housing is pulled out of the catheter hub, the safety protection cover is separated from the catheter hub, and the tail end cap is connected to the tail end of the catheter hub in a sealed manner.

### BRIEF DESCRIPTION OF THE DRAWINGS

FIG. 1 is a schematic external view of an indwelling needle provided by an embodiment of the present application;
FIG. 2 is an exploded view of the indwelling needle provided by an embodiment of the present application;
FIG. 3 is a schematic split view of a needle tip protection housing in the indwelling needle provided by an embodiment of the present application;
FIG. 4 is a sectional view of the indwelling needle in a factory default state provided by an embodiment of the present application;
FIG. 5 is a sectional view of the indwelling needle in a needle withdrawn state provided by an embodiment of the present application; and
FIG. 6 is an enlarged view of Part A in FIG. 4.

In drawings, reference symbols represent the following components:
1-catheter hub; 11-catheter; 12-upper dosing port; 13- positioning groove;
2-needle hub; 21-needle tubing;
3-safety protection cover;
4-vent plug;
5-tail end cap; 51-plunger;
6-needle tip protection housing; 61-anti-puncture plug; 62-hook portion; 63-elastic cannula; 64-limiting clamp; 65-isolation plug; 66-clamp head; 67-groove; 68-slot;
7-dosing port sealing pipe; and
8-upper dosing port protection cap.

### DETAILED DESCRIPTION OF THE INVENTION

Considering that there is a risk of accidental contact with a needle tip after a puncture needle is withdrawn, which may cause infections to outsiders, some indwelling needle structures will add needle tip protection components. When the needle is withdrawn, the needle tip protection component wraps the needle tip and is taken out together with a needle core to shield the needle tip.

However, the needle tip protection component is usually a common elastic sheet structure that locks the needle tip. A chamber where the needle tip protection component is located is communicated with a puncture opening on the human body and is prone to impregnation by blood. As a result, when the needle is withdrawn, the removed needle tip protection component is stained with the patient's blood, resulting in bacterial spillage, infectious disease infections and other hazards. Since most open-type indwelling needles cannot avoid blood leakage during operation, improvement is urgently needed.

In view of the above situation, an embodiment of the present application provides an indwelling needle.

A description will be made in detail to the embodiments of the present application, examples of which are illustrated in the accompanying drawings. The reference symbols which are the same or similar throughout the accompanying drawings represent the same or similar components or components with the same or similar functions. The embodiments described below with reference to the accompanying drawings are exemplary, and are intended to explain the present application, rather than being construed as limitations to the present application.

In the description of the present application, unless otherwise definitely specified and limited, the terms "connected with each other", "connected to/with", "fixed", and the like need to be broadly understood. For example, connection may be fixed connection, or detachable connection; or may be mechanical connection, or electrical connection; or may be indirect connection via an intermediation, or communication of inner parts of two elements, or an interaction relationship between two elements. For those of ordinary skill in the art, specific meanings of the foregoing terms in the present application may be understood based on specific situations.

In the description of the present application, unless otherwise definitely specified and limited, a first feature being provided "above" or "below" a second feature may mean that the first feature is in direct contact with the second feature, or mean that the first and second features are not in direct contact, but via other additional feature therebetween. Moreover, the first feature being provided "over", "above", and "on" the second feature may mean that the first feature is provided directly above or diagonally above the second feature, or merely means that a level of the first feature is higher than the second feature. The first feature being provided "under", "below", and "beneath" the second feature may mean that the first feature is provided directly below or diagonally below the second feature, or merely means that a level of the first feature is lower than the second feature. The technical solution of the present application will be further described in detail below in conjunction with the accompanying drawings and the specific embodiments.

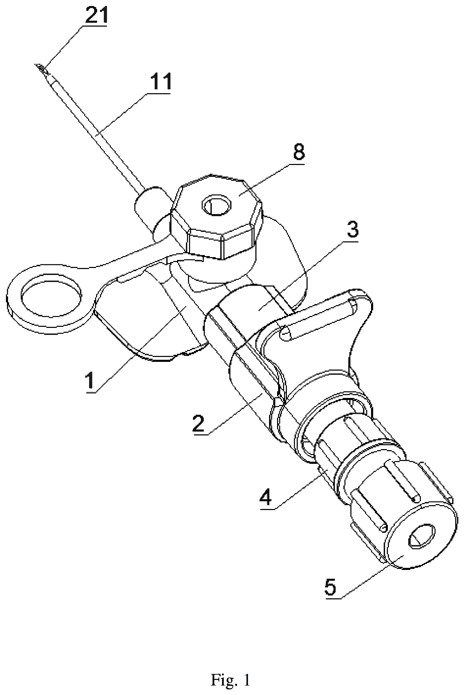

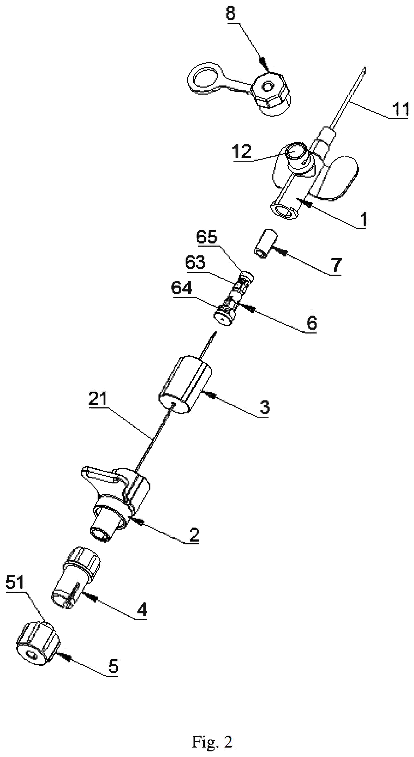

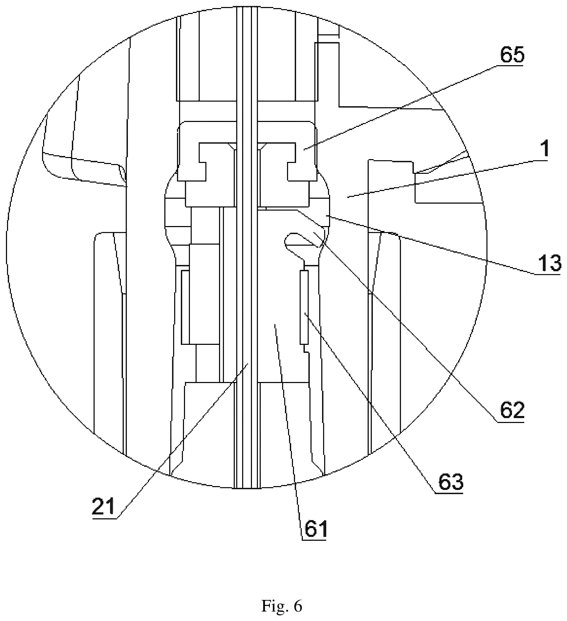

Referring to FIGS. 1 to 6, the present embodiment provides an indwelling needle. The indwelling needle includes a catheter hub 1, a needle hub 2 and a safety protection cover 3.

A catheter 11 is provided at a head end of the catheter hub 1, and an opening is provided in a tail end of the catheter hub 1; a needle tubing 21 is provided at a head end of the needle hub 2, and a vent plug 4 and a tail end cap 5 are provided at a tail end of the needle hub 2; a needle tip protection housing 6 is provided in the safety protection cover 3, and an isolation plug 65 is provided at a head end of the needle tip protection housing 6.

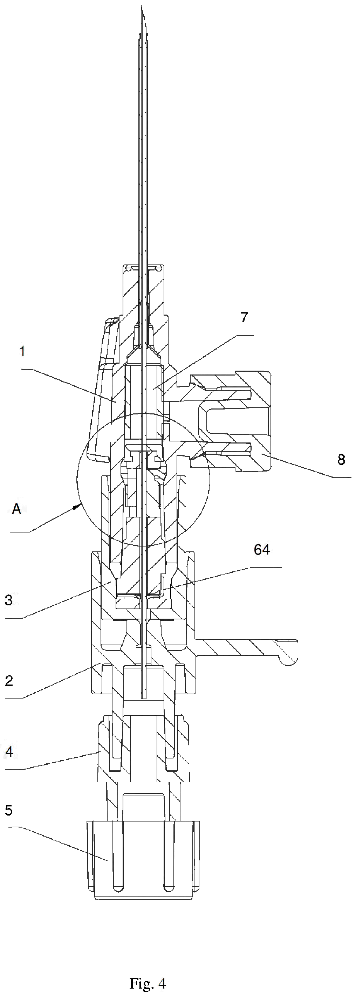

In a factory default state, as shown in FIG. 4, the safety protection cover 3 is sleeved at the tail end of the catheter hub 1, the needle hub 2 is sleeved on the safety protection cover 3, the needle tip protection housing 6 is inserted into the catheter hub 1, an inner cavity of the catheter hub 1 is sealed by means of the isolation plug 65, and the needle tubing 21 passes through the needle tip protection housing 6 and then extends into the catheter 11. For example, the catheter hub 1, the safety protection cover 3, the needle hub 2, the vent plug 4 and the tail end cap 5 are sequentially and detachably connected in a plug-in manner; and the catheter hub 1, the safety protection cover 3 and the needle hub 2 can be easily operated and unplugged.

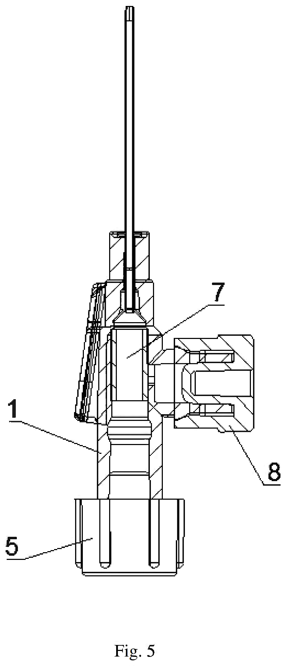

In a needle withdrawn state, as shown in FIG. 5, a needle tip of the needle tubing 21 is withdrawn into the needle tip protection housing 6, the needle tip protection housing 6 is pulled out of the catheter hub 1, the safety protection cover 3 is separated from the catheter hub 1, and the tail end cap 5 is connected to the tail end of the catheter hub 1 in a sealed manner.

In one embodiment, a plunger 51 which extends into the catheter hub 1 is arranged on the tail end cap 5, and the tail end cap 5 is in threaded fit with the tail end of the catheter hub 1.

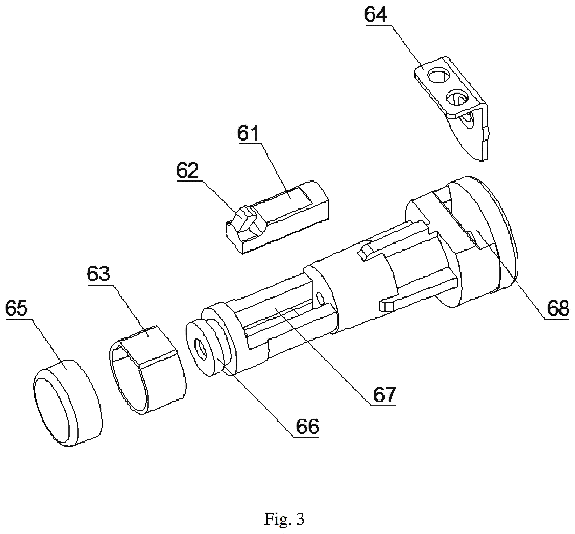

As shown in FIG. 3, a chuck 66 is provided at the head end of the needle tip protection housing 6 here, the isolation plug 65 is an elastomer (e.g., colloidal silica) and is hooped on the chuck 66, and the isolation plug 65 is in interference fit with a wall of the inner cavity of the catheter hub 1. Therefore, in the case that the safety protection cover 3 is not separated from the catheter hub 1, the inner cavity of the catheter hub 1 can ensure a sealed state, preventing blood from penetrating to rear components and contaminating the entire needle tip protection housing 6.

In one embodiment, the needle tip protection housing 6 is provided with a groove 67, and an anti-puncture plug 61 is embedded into the groove 67; in the factory default state, a lower surface of the anti-puncture plug 61 props against the needle tubing 21; and in the needle withdrawn state, the anti-puncture plug 61 sinks into the groove 67 and blocks a passage path of the needle tubing 21. Therefore, when the needle tip is withdrawn into the anti-puncture plug 61 and falls, the anti-puncture plug 61 effectively prevents the needle tubing 21 from being reinserted, thereby reducing the risk of infections.

In one embodiment, a hook portion 62 protrudes from an outer surface of the anti-puncture plug 61, and an inner wall of the tail end of the catheter hub 1 is provided with a positioning groove 13; in the factory default state, the hook portion 62 extends into the positioning groove 13 to limit an axial movement of the needle tip protection housing 6; and in the needle withdrawn state, the hook portion 62 escapes from the positioning groove 13 as the anti-puncture plug 61 falls, such that the needle tip protection housing 6 can move freely in an axial direction. Therefore, in the factory default state, the indwelling needle has a stable structure; and in the needle withdrawn state, the needle tip protection housing 6 escapes smoothly from the catheter hub 1.

In one embodiment, an elastic cannula 63 that exerts a pressure on the outer side of the anti-puncture plug 61 is sleeved on the needle tip protection housing 6, so as to avoid the anti-puncture plug 61 from escaping from the groove and to ensure that the anti-puncture plug 61 accurately performs a sinking action.

In one embodiment, a slot 68 is provided in the tail end of the needle tip protection housing 6, a limiting clamp 64 is inserted into the slot 68, and the limiting clamp 64 is used to prevent the needle tip of the needle tubing 21 from escaping. With the needle withdrawal action, the needle tip is avoided from being exposed, and the safety protection cover 3 can be driven to be separated from the catheter hub 1.

It is also worth mentioning that the safety protection cover 3 not only wraps the needle tip protection housing 6, but also the opening at the tail end of the catheter hub 1. When the isolation plug 65 is pulled out of the catheter hub 1, the safety protection cover 3 will have a blocking effect in the case of blood leakage, such that an operator will not be in a hurry, and a larger needle tip protection structure is more secure to use.

In one embodiment, the catheter hub 1 is provided with an upper dosing port 12, and an upper dosing port protection cap 8 is installed on the upper dosing port 12 and can be disassembled and assembled in use, thereby ensuring the cleanliness of the upper dosing port 12.

In one embodiment, a dosing port sealing pipe 7 is provided in the inner cavity of the catheter hub 1, and the upper dosing port 12 is sealed by means of the dosing port sealing pipe 7; and during dosing, a dosing pressure causes the dosing port sealing pipe 7 made of a silica gel material to deform, making the upper dosing port 12 unblocked. In addition, for the requirements of drug liquid filtration, a filter element and other components may also be provided in the inner cavity of the catheter hub 1.

In summary, the above-mentioned indwelling needle is equipped with the needle tip protection housing having a special structure, which not only reliably seals a relevant chamber of a puncture opening on the human body, but also forms a reliable protection for the needle tip of the needle tubing, thereby effectively avoiding external infections to patients caused by blood spillage, reducing the risk of needle puncture, and improving the convenience and safety in the use of the indwelling needle.

There are also various variations and changes in the embodiments of the present application, all of which fall within the protection scope of the present application.

## Claims

1. An indwelling needle, comprising a catheter hub (1) and a needle hub (2), wherein a catheter (11) is provided at a head end of the catheter hub (1), and an opening is provided in a tail end of the catheter hub (1); a needle tubing (21) is provided at a head end of the needle hub (2), and a vent plug (4) and a tail end cap (5) are provided at a tail end of the needle hub (2);
the indwelling needle further comprising a safety protection cover (3), wherein a needle tip protection housing (6) is provided in the safety protection cover (3), and an isolation plug (65) is provided at a head end of the needle tip protection housing (6);
in a factory default state, the safety protection cover (3) is sleeved at the tail end of the catheter hub (1), the needle hub (2) is sleeved on the safety protection cover (3), the needle tip protection housing (6) is inserted into the catheter hub (1), an inner cavity of the catheter hub (1) is sealed by means of the isolation plug (65), and the needle tubing (21) passes through the needle tip protection housing (6) and then extends into the catheter (11); and
in a needle withdrawn state, a needle tip of the needle tubing (21) is withdrawn into the needle tip protection housing (6), the needle tip protection housing (6) is pulled out of the catheter hub (1), the safety protection cover (3) is separated from the catheter hub (1), and the tail end cap (5) is connected to the tail end of the catheter hub (1) in a sealed manner.

2. The indwelling needle according to claim 1, wherein a chuck (66) is provided at the head end of the needle tip protection housing (6), the isolation plug (65) is an elastomer and is hooped on the chuck (66), and the isolation plug (65) is in interference fit with a wall of the inner cavity of the catheter hub (1).

3. The indwelling needle according to claim 1, wherein the needle tip protection housing (6) is provided with a groove (67), and an anti-puncture plug (61) is embedded into the groove (67); in the factory default state, a lower surface of the anti-puncture plug (61) props against the needle tubing (21); and in the needle withdrawn state, the anti-puncture plug (61) sinks into the groove (67) and blocks a passage path of the needle tubing (21).

4. The indwelling needle according to claim 3, wherein a hook portion (62) protrudes from an outer surface of the anti-puncture plug (61), and an inner wall of the tail end of the catheter hub (1) is provided with a positioning groove (13); in the factory default state, the hook portion (62) extends into the positioning groove (13) to limit an axial movement of the needle tip protection housing (6); and in the needle withdrawn state, the hook portion (62) escapes from the positioning groove (13) as the anti-puncture plug (61) falls, such that the needle tip protection housing (6) can move freely in an axial direction.

5. The indwelling needle according to claim 3, wherein an elastic cannula (63) that exerts a pressure on the outer side of the anti-puncture plug (61) is sleeved on the needle tip protection housing (6).

6. The indwelling needle according to claim 1, wherein a slot (68) is provided in the tail end of the needle tip protection housing (6), a limiting clamp (64) is inserted into the slot (68), and the limiting clamp (64) is used to prevent the needle tip of the needle tubing (21) from escaping.

7. The indwelling needle according to claim 1, wherein the catheter hub (1) is provided with an upper dosing port (12), and an upper dosing port protection cap (8) is installed on the upper dosing port (12).

8. The indwelling needle according to claim 7, wherein a dosing port sealing pipe (7) is provided in the inner cavity of the catheter hub (1), and the upper dosing port (12) is sealed by means of the dosing port sealing pipe (7); and during dosing, a dosing pressure causes the dosing port sealing pipe (7) to deform, making the upper dosing port (12) unblocked.

9. The indwelling needle according to claim 1, wherein a plunger (51) which extends into the catheter hub (1) is arranged on the tail end cap (5), and the tail end cap (5) is in threaded fit with the tail end of the catheter hub (1).

10. The indwelling needle according to claim 1, wherein in the factory default state, the catheter hub (1), the safety protection cover (3), the needle hub (2), the vent plug (4) and the tail end cap (5) are sequentially and detachably connected in a plug-in manner.
